# EUROPEAN PATENT SPECIFICATION

(11) **EP 4 034 182 B1**
(45) Date of publication and mention of the grant of the patent: **28.06.2023**
(21) Application number: 20785456.3
(22) Date of filing: 24.09.2020
(51) Int. Cl.: A61M 1/00, A61M 25/00

(54) **ABSCESS DRAINAGE SYSTEM**
SYSTEM ZUR DRAINAGE VON ABSZESSEN
SYSTÈME DE DRAINAGE D'ABCÈS

(30) Priority: 27.09.2019 SE 1951099
(43) Date of publication of application: 03.08.2022
(73) Proprietor: HELSE STAVANGER HF, 4068 STAVANGER (NO)
(72) Inventor: THOLFSEN, Tore, 4068 Stavanger (NO); NESVÅG, Sverre, 4068 Stavanger (NO)
(74) Representative: Brann AB
(86) International application number: PCT/EP2020/076782
(87) International publication number: WO 2021/058681

(56) References cited:
- CN-A- 106 994 191
- US-A1- 2005 085 769
- US-B2- 9 358 328

## Description

### TECHNICAL FIELD

The present invention relates to a system and device for drainage of any pathological fluid filled spaces within the body, e.g. abscesses, bilioma, hematoma, leakage from the gastrointestinal organs, necrotic tissue, contained fluid after surgery, etc., and a kit comprising the device.

### BACKGROUND

The present invention relates to a system and device for drainage of any pathological fluid filled spaces within the body, e.g. abscesses, bilioma, hematoma, leakage from the gastrointestinal organs, necrotic tissue, contained fluid after surgery, etc., and a kit comprising the device.

Most frequently the fluid to be drained is comprised in an abscess and for the simplicity in the following description the fluid filled space will be referred to as an abscess.

An abscess is a pocket of pus in tissue of the body usually triggered by a bacterial infection. Abscess drainage is the treatment typically used to clear an abscess of pus to initiate the healing process and accelerate the recovery of the patient.

The treatment of abscesses is very important because untreated infections carry a high morbidity and mortality if they are not treated properly. The classic treatment of abscesses used to be surgical drainage, in the last decades due to the advances of radiological intervention, the use of abscessography, ultrasonography (US) and computed tomography (CT) has led to better diagnosis, and to the percutaneous image-guided catheter drainage of abscesses and fluid collections. This is now a well-established and acceptable alternative to surgery in many cases. Abscesses require complete evacuation and often continuous external drainage prevent the dissemination of the infection.

The drainage catheters may be inserted under sonographic, CT or fluoroscopic guidance. There is a wide selection of commercially available catheters normally ranging in size from 8 to 14 French and they can be made of different materials. Two different techniques are used for the insertion of the drain into the fluid collections, either the Seldinger technique in which the fluid collection is punctured with a small needle or a needle-sheath combination, and then a guide-wire is passed through the needle and into the collection. The needle is removed and a series of dilators is passed over the wire followed by the drainage catheter. The alternative method is the trocar catheter system which has a drainage catheter sheathed over a sharpened stylet or trocar. The catheter is inserted in a single pass over the same route as in the preceding diagnostic needle aspiration.

Once the catheter has been adequately positioned, the collection is evacuated and the cavity is gently irrigated with sterile saline. Effective irrigation may require many liters of saline and can last for days and weeks. The catheter is typically secured in position and attached to a drainage apparatus such as a drainage bag or suction. The patient and catheter are followed-up with regard to temperature, biochemical infectious parameters and the amount of daily drainage. Periodic or continuous irrigation of the catheter with saline is often necessary to ensure the patency of the catheter lumen.

Unfortunately today's method of abscess drainage and the catheters applied introduce complications. One of the most common complications is that the drain catheter clogs up or kink so often it has to be removed and unclogged, and/or repositioned. Also, if a drain catheter has been removed and unclogged, or repositioned, a number of times, it will eventually have to be changed to a new one. Alternatively, or in combination, multiple drains must sometimes be inserted, which is of course expensive and time consuming as well as cumbersome for the patient.

There are substantial problems with using existing solutions wherein the same catheter lumen is used for both insertion of rinsing fluid and removal of abscess fluid. Firstly, the needed length of the drain from the abscess to the connector for inserting the rinsing fluid makes it necessary to insert quite a lot of fluid just to reach the abscess. As the drain itself is not empty before inserting the fluid, this will result in an unwanted pressure increase in the abscess. It also means that the fluid reaching the abscess is a mix of "old polluted" fluid and "new" clean fluid, making the rinsing process less effective. The only way the rinsed fluids can come out again is by a decrease of the (unwanted) pressure in the abscess. The result is that a mixture of clean and polluted fluid runs back and forth in the same tube, only slowly, and inefficiently, rinsing the abscess.

A further problem is that it is common that the catheter dislocates and the patient has to go through a new intervention, which is time consuming, labor intensive and expensive for the hospitals.

Another major problem with the catheters and abscess drainage methods used today is that the patient often needs to be hospitalized during the abscess drainage process, due to the necessity to maintain the functionality and irrigation of the drainage system by monitoring and possible removal, unclogging, repositioning, and/or changing of the catheter performed by medical staff. This is of course also expensive and time consuming for the hospital, as well as and cumbersome for the patient.

Yet another problem is that it is not possible during the process to see what is going on inside the abscess. Therefore, the rinsing/cleansing performed is in many cases inefficient. If the abscess is inadequately rinsed/cleansed, this leads to the further problem that the abscess is likely to recur.

One example of related art is found in US 2005/085769 A1, which discloses a device with first and second lumens for inserting fluid into and suck fluid from a body cavity or conduit, respectively. The lumens are configured to be connected to a pump system. The distal tip of the catheter has openings, allowing fluid to be inserted into the body cavity or conduit.

It is clear that improved solutions for draining an abscess are needed.

### SUMMARY

The invention is defined by the features of independent claim 1.

The present invention relates to a system and device for drainage of any pathological fluid filled spaces within the body, e.g. abscesses, bilioma, hematoma, leakage from the gastrointestinal organs, necrotic tissue, contained fluid after surgery, etc., and a kit comprising the device.

Most frequently the fluid to be drained is comprised in an abscess and for the simplicity in the following description the pathological fluid filled space will be referred to as an abscess. However, it is evident to a person skilled in the art that systems and devices according to embodiments presented herein may advantageously be used for draining any pathological fluid filled space within the body. The object of the present invention is to eliminate or at least to minimize the problems discussed above. This is achieved by a catheter system and an abscess draining device according to the appended independent claims. Advantageous embodiments are presented in the appended dependent claims.

In a first aspect of the invention, there is provided a catheter system comprising first and second lumens, the first lumen being configured to transport rinsing fluid into an abscess and the second lumen being configured to transport abscess fluid out from the abscess. A respective proximal end of the first and second lumens are configured to be connected to a pump system so as to enable the pump system to pump the rinsing fluid into the abscess via an overpressure applied to the first lumen, and further enable the pump to suck abscess fluid out from the abscess via an under pressure applied to the second lumen. The catheter system further comprises a first nozzle head attached to a distal end of the first lumen, which first nozzle head is configured to cause the rinsing fluid exiting from the first lumen to be distributed in the abscess in response to movements of the first nozzle head resulting from the rinsing fluid flowing through the first nozzle head.

Suitably, the catheter system is thereby enabled to effectively rinse the abscess as the rinsing fluid advantageously reaches every part of the abscess without manual repositioning of the catheter system, due to the system being configured to cause rinsing fluid exiting from the first lumen to be distributed in the abscess in response to movements of the nozzle head resulting from the rinsing fluid flowing through the nozzle head. A further advantage linked to the effective cleansing is that the risk that the abscess recurs is greatly reduced, thereby reducing the time and cost spent on aftercare, contributes to reducing the use of antibiotics and makes the patient experience significantly better.

Another advantage is the catheter system advantageously reduces or even eliminates the need for hospital staff to flush/irrigate the catheter system once the system is established and running, because the double lumens greatly reduce the risk that the first lumen is clogged. Thereby, a patient can be treated at home, or at the hospital with much less supervision and assistance, which saves time and money for the hospitals, as well as makes the experience less cumbersome for the patient.

Also, if medical staff is assisting the patient, the rate of success of the draining is increased, since it is much less dependent on the experience of the medical staff then when one of the previously known solutions with manual repositioning within the abscess and/or frequent removal and cleansing or changing of the catheter system is required.

Yet a further advantage is that it is possible to achieve a continuous and effective rinsing of the abscess without increasing the pressure in the abscess, and also where it is possible to dissolve blocked pus in the tube with a combination of pressure (over pressure) and vacuum (under pressure).

The first nozzle head is tiltable around an axis being orthogonal to a symmetry axis of the first lumen. The first nozzle head may be rotatable around an axis being parallel to a symmetry axis of the first lumen. The first nozzle head may be bendable, for example by comprising a flexible material, and possibly being elongated, and/or comprising one or more flexible section. These and other alternative configurations for enabling the first nozzle head to cause the rinsing fluid exiting from the first lumen to be distributed in the abscess in response to movements of the first nozzle head resulting from the rinsing fluid flowing through the first nozzle head all contribute to the advantage of effectively rinsing the abscess, by the rinsing fluid advantageously reaching different parts, preferably every part, of the abscess without manual repositioning of the catheter system.

In one or more embodiment, the first nozzle head comprises at least one output opening configured to eject the rinsing fluid, which at least one output opening is arranged off centered with respect to the symmetry axis of the first lumen. Alternatively, or in combination, the first nozzle head may comprise at least two output openings configured to eject the rinsing fluid, wherein a first output opening of said at least two output openings is of a first size and a second output opening of said at least two output openings is of a second size different from the first size. Suitably, the first nozzle head will according to any of these embodiments be caused to move within the abscess due to fluid being ejected asymmetrically through the at least one opening, thereby further contributing to effectively rinsing the abscess, by the rinsing fluid advantageously reaching different parts, preferably every part, of the abscess without manual repositioning of the catheter system.

In one or more embodiments, the first lumen may be comprised in the second lumen. In some of these embodiments, the second lumen may be configured to receive the first lumen and the first nozzle head while the second lumen is in place inside the abscess. Thereby, the need to remove the entire catheter system to remove a clog or the like is eliminated or at least greatly reduced.

In some embodiments, the second lumen may be configured to receive and transport at least one of a tool and a cannula for deployment in the abscess.

The catheter system may be connected to at least one of an internal fixation device and an external fixation device. Due to the fixation, the risk of the catheter system or any part of it dislodging during use is advantageously greatly reduced. Furthermore, possibilities to decrease the necessity of several radiological procedures are introduced due to the better fixation of the catheter system, since the tube-in-tube system firstly renders any clogging problem less likely to happen, and secondly, if clogging occurs, the inner tube can be changed without the assistance of a radiologist. The abscess drainage will thereby be more efficient and the need of multiple drains is eliminated or at least greatly reduced.

In a second aspect of the invention, there is provided an abscess draining device comprising a catheter system according to any of the embodiments presented herein, and a pump system connected to a respective proximal end of the first and second lumens, the pump system being configured to pump the rinsing fluid into the abscess via an applied overpressure and suck abscess fluid out from the abscess via an applied under pressure.

The effects and/or advantages presented in the present disclosure for embodiments of the catheter system according to the first aspect may also apply to corresponding embodiments of the abscess draining device.

In a third aspect of the invention, there is provided a kit for abscess draining comprising a catheter system according to any of the embodiments presented herein, and a kit package configured to enclose the catheter system. The kit may further comprise a pump system connected to a respective proximal end of the first and second lumens, the pump system being configured to pump the rinsing fluid into the abscess via an applied overpressure and suck abscess fluid out from the abscess via an applied under pressure, wherein the kit package is further configured to enclose the pump system.

Many additional benefits and advantages of the present invention will be readily understood by the skilled person in view of the detailed description below.

It is noted that embodiments of the present disclosure relate to all possible combinations of features recited in the claims.

### DRAWINGS

The invention will now be described in more detail with reference to the appended drawings, wherein
Fig. 1 is a schematic overview of a catheter system, according to one or more embodiments;
Fig. 2 is a schematic overview of a catheter system including a nozzle head, according to one or more embodiments;
Fig. 3A and B are schematic overviews of alternative embodiments of catheter systems, from a side view and in cross section;
Fig. 4 is a schematic overview of an abscess draining device, according to one or more embodiments;
Fig. 5 illustrates a catheter system with external fixation means for fixating the catheter system to the skin of a patient and internal fixation means for fixating the catheter system inside an abscess, according to one or more embodiments; and
Figs. 6A to 6C are schematic overviews of alternative embodiments of the nozzle head, according to embodiments.

All the figures are schematic, not necessarily to scale, and generally only show parts which are necessary in order to elucidate the respective embodiments, whereas other parts may be omitted or merely suggested. Any reference number appearing in multiple drawings refers to the same object or feature throughout the drawings, unless otherwise indicated.

### DETAILED DESCRIPTION

### Introduction

Aspects of the present disclosure will be described more fully hereinafter with reference to the accompanying drawings. The devices and method disclosed herein can, however, be realized in many different forms and should not be construed as being limited to the aspects set forth herein. Like numbers in the drawings refer to like elements throughout.

The terminology used herein is for the purpose of describing particular aspects of the disclosure only, and is not intended to limit the invention. As used herein, the singular forms "a", "an" and "the" are intended to include the plural forms as well, unless the context clearly indicates otherwise.

Today's abscess draining techniques have changed little over the last decades. Typically when the physicians find it necessary to do a drainage the patient is hospitalized and send from the bed ward to the radiology department where the radiologist do the intervention with insertion of the drain either by the "Seldinger" technique or the "trochar" technique under guidance of ultrasound or x-rays (e.g. CT/fluoroscopy). When access to the fluid collection is achieved, fluid is aspirated by a syringe and the drain is inserted and fixated (usually by tighten the "pig-tail" on the drain and tape to the skin). After the procedure the patient goes back to the ward and the nursing staff takes care of the drain that usually needs to be flushed regularly and the abscess cavity rinsed up to 4-6 times a day until the infection clears up. Often antibiotic treatment is started simultaneously due to suspicion of bacteremia. The patients are usually admitted during this treatment, some are discharged with the drain, but then they are often in the need of daily supervision by a home nursing service several times a day. Rinsing and clearing of such fluid filled spaces can be quite time consuming and last for several weeks, even months. There are three main problems with the known solutions. The first is related to the principle of the system itself. The needed length of the drain from the abscess to the connector for inserting the rinsing fluid makes it necessary to insert quite a lot of fluid just to reach the abscess. As the drain itself is not empty before inserting the fluid, this will result in an unwanted pressure increase in the abscess. It also means that the fluid reaching the abscess is a mix of "old polluted" fluid and "new" clean fluid, making the rinsing process less effective. The only way the rinsed fluids can come out again is by a reducing the (unwanted) pressure in the abscess. The result is that a mixture of clean and polluted fluid runs back and forth in the same tube, only slowly, and inefficiently, rinsing the abscess. A second problem is related to the nozzle head inside the tube. The conventional "pig tail" have several holes for the rinsing fluid to spread into the abscess. As the "pig tail" has a fixed position in the abscess, it only reaches the nearest area and as the "rinsed" fluid has to return in through the same holes, whereby the rinsing effect is poor. These two problems are why the rinsing process can take weeks and sometimes months. The main problem is not the healing process of the abscess but the low effectiveness of the rinsing system itself. In addition there is a third common problem with the conventional drains are that they often clog due to debris, dislocate or there are significant problems with leakage, which means re-admittance to the hospital and additional intervention at the radiology department must be done. Often multiple drains insertions are necessary.

Embodiments herein provide a catheter system that is highly effective for both draining and irrigation of any type of fluid collections in an abscess, by using a double lumen catheter, one lumen for drainage and one lumen for irrigation, in combination with a moveable nozzle head. The catheter system according to embodiments presented herein further enable abscess draining and irrigation without, or with a minimum of, manual intervention once the draining process has been initiated. Furthermore, the nozzle head of embodiments described herein is advantageously structured to reach every part of the abscess without manual repositioning of the catheter system, due to it being configured to cause rinsing fluid exiting from the first lumen to be distributed in the abscess in response to movements of the nozzle head resulting from the rinsing fluid flowing through the nozzle head. This, and other advantageous features, is further described below in connection with the figures.

Embodiments of the present invention can be applied in all patients admitted to hospital that are in need of drainage of any fluid collections contained in the soft tissue of the body, e.g. hematoma, abscesses, pseudo cysts, bilioma, etc.

Due to the ease of using the inventive system and device, i.e. since this system is fully automatic and not dependent on hospital staff to flush/irrigate the catheter once the system is established and running, a patient can be treated at home, or at the hospital with much less supervision and assistance, which saves time and money for the hospitals. Also, if medical staff is assisting the patient, the success of the draining is much less dependent on the experience of the medical staff then when one of the previously known solutions is used.

Embodiments presented herein hence describe a medically- and cost-effective system for drainage of abscesses, based on the principle were the flushing fluid is inserted in an inner tube while the returning pus/fluid flows back through an outer tube, which may also be referred to herein as an outer casing or a casing catheter. This is a system where it is possible to achieve a continuous effective rinsing of the abscess without increasing the pressure in the abscess and also where it is possible to dissolve blocked pus in the tube with a combination of pressure (over pressure) and vacuum (under pressure) or, according to some embodiments, enabling extracting the inner tube, insert more cleansing fluid and then reinsert the inner tube without moving the outer tube. The outer tube may also in some embodiments be used to insert other equipment into the abscess, e.g. for cutting/removing dead tissue or for inserting antibiotics, enzymes (for dissolving solid pus) or other medication.

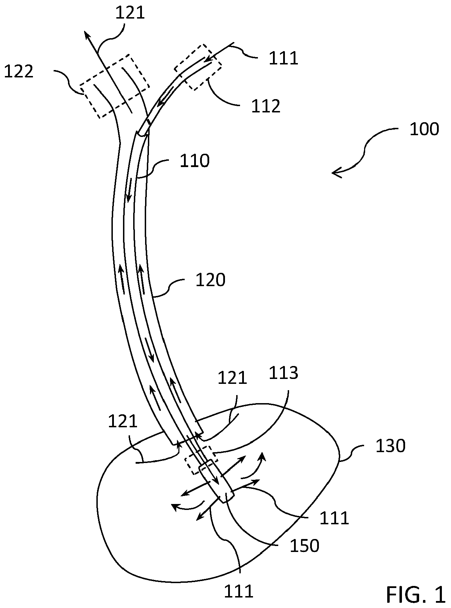

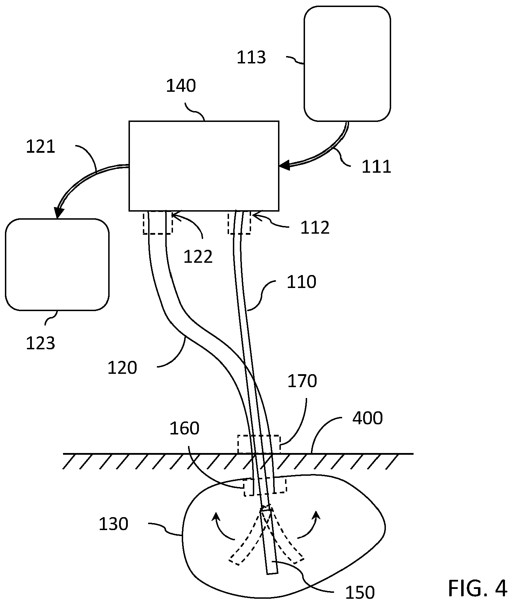

Turning first to **Fig. 1**, there is shown a catheter system 100 comprising a first lumen 110 and a second lumen 120, the first lumen 110 being configured to transport rinsing fluid 111 into an abscess 130 and the second lumen 120 being configured to transport abscess fluid 121 out from the abscess 130. A respective proximal end 112 of the first lumen 110, and a proximal end 122 of the second lumen 120, respectively, is configured to be connected to a pump system 140, as illustrated in **Fig. 4****,** so as to enable the pump system 140 to pump the rinsing fluid 111 into the abscess 130 via an overpressure applied to the first lumen 110 and further enable the pump to suck abscess fluid 121 out from the abscess 130 via an under pressure applied to the second lumen 120. The catheter system 100 further comprises a first nozzle head 150 attached to a distal end 113 of the first lumen 110. The first nozzle head 150 is configured to cause the rinsing fluid 111 exiting from the first lumen 110 to be distributed in the abscess 130 in response to movements of the first nozzle head 150 resulting from the rinsing fluid 111 flowing through the first nozzle head 150.

Suitably, the nozzle head 150 being automatically moveable within the abscess by the flow of the rinsing fluid 111, the movements of the nozzle head being comparable to it being propelled by a "watcr-jct" effect, enables efficient cleansing of all parts of the abscess 130. As described herein, this movement is not dependent of an operator. This solves, or at least ameliorates to a large degree, the well-known problem of not reaching all parts of an abscess using previous abscess draining solutions that have fixed nozzle heads or other fluid outlets. The efficient rinsing in turn leads to a reduced risk of the abscess reappearing compared to using previously known abscess draining solutions.

Furthermore, the nozzle head 150 being automatically moveable within the abscess 130 solves the need for medical staff to reposition the catheter system during draining to try to reach different parts of an abscess. This in turn leads to the substantial benefit of reducing or even completely eliminating the need for manual intervention during operation, with maintained or even improved drainage and irrigation result. The system with the nozzle head 150 is designed to give a better rinsing effect of the abscess and the double lumen catheter will optimize the flow of fluid in and out of the abscess. Better rinsing advantageously contributes to lesser use of antibiotics, faster recovery and less time spent in hospital for the patient. Consequently, embodiments herein optimize and streamline the treatment of all unwanted localized fluid collections in the body, decrease the need for hospitalization of the patients, decrease the work load for the nursing staff on the bed wards and decrease the necessity of multiple procedures at for example the radiology department. Furthermore, embodiments herein contribute to decreasing the use of antibiotics, as the risk of the infection, and hence the abscess, reappearing is reduced.

According to the claimed invention, the first nozzle head 150 is tiltable around an axis being orthogonal to a symmetry axis A of the first lumen 110. In combination, the first nozzle head 150 may be rotatable around an axis being parallel to a symmetry axis A of the first lumen 110.

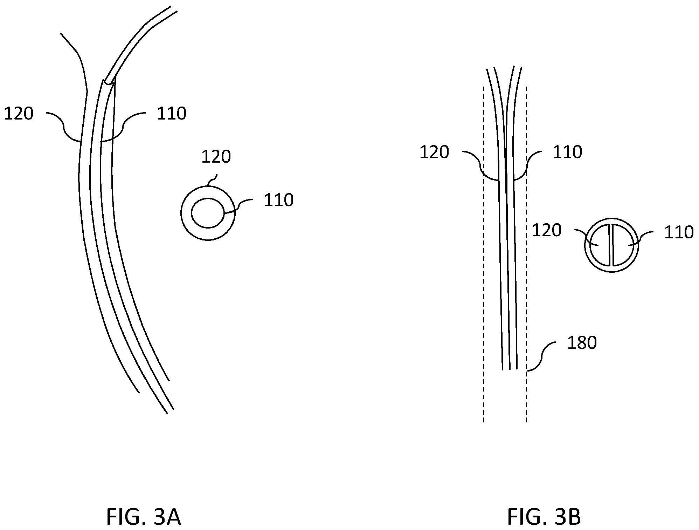

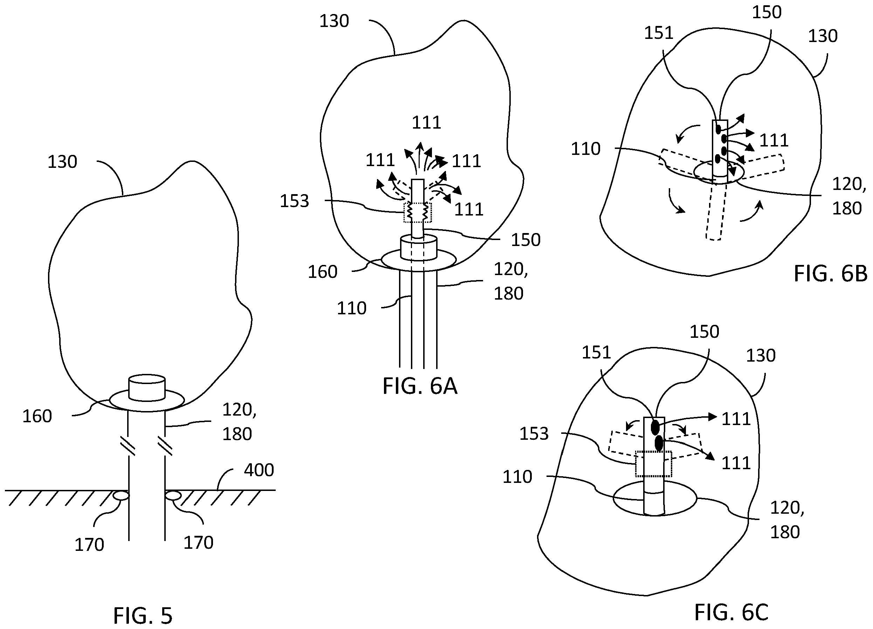

The first nozzle head 150 may be attached to the distal end 113 of the first lumen 110 in any suitable manner. In some embodiments, the first nozzle head 150 may be tiltable and/or rotatable in relation to the distal end 113 of the first lumen by means of a joint 152 or other connecting means connecting the distal end 113 of the first lumen 110 to the first nozzle head 150 and being configured to enable tilting and optionally rotation between the distal end 113 of the first lumen 110 to the first nozzle head 150. In **Fig. 6B** it is schematically illustrated from a "top view" (looking at an angle into the proximal end of the second lumen 120 or casing catheter 180, further described in connection with Fig. 3B) how the nozzle head 150 may move within the abscess 130 during operation. This is illustrated by the dashed nozzle heads in different positions and the adjacent curved arrows, indicating rotation and/or tilting of the first nozzle head 150 in relation to the first lumen 110.

Further to the first nozzle head 150 being tiltable and optionally rotatable in relation to the distal end 113 of the first lumen, the first nozzle head 150 may comprise a flexible material, thereby being configured to bend in response to the rinsing fluid 111 flowing through the first nozzle head 150. This is illustrated by the dashed, bent, nozzle heads in different positions and the adjacent curved arrows in **Fig. 4**.

In the embodiments wherein the first nozzle head 150 comprises a flexible material, the entire first nozzle head 150, or a part of it, may be made of the flexible material, as exemplified in Fig. 4. Alternatively, the first nozzle head 150 may comprise at least one flexible section 153 as illustrated in an exemplary, non-limiting, manner in the side view of **Fig. 6A** and the "top view" of **Fig. 6C** (looking at an angle into the proximal end of the second lumen 120 or casing catheter 180, further described in connection with Fig. 3B). Each of the at least one flexible section 153 may comprise a flexible material and/or be structured in a manner allowing it to bend, tilt and/or rotate, thereby moveably connecting the parts of the first nozzle head 150 that extend distally and proximally, respectively, from the flexible section 153. According to embodiments wherein the first nozzle head 150 comprises one or more flexible section 153, the first nozzle head 150 is configured to cause the rinsing fluid 111 exiting from the first lumen 110 to be distributed in the abscess 130 in response to movements of the one or more flexible section 153 of the first nozzle head 150, and consequently movements of any part distal to one or more flexible part 153, resulting from the rinsing fluid 111 flowing through the first nozzle head 150.

The first nozzle head 150 may in any of these embodiments be elongated in shape, which makes it easier for it to bend, tilt and/or rotate at one or more locations along its longitudinal axis, and also to reach further parts of the abscess.

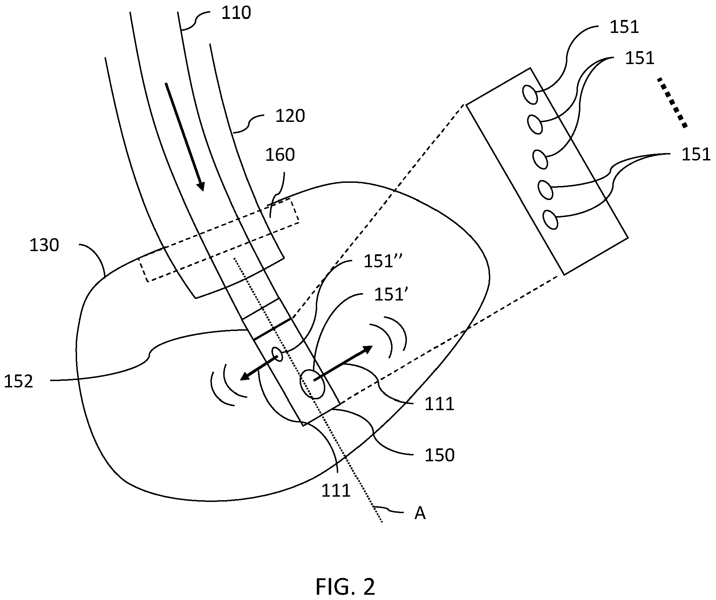

As illustrated in the embodiments shown in **Figs. 2****,** **6B and 6C**, the first nozzle head 150 may comprise at least one output opening 151 configured to eject the rinsing fluid 111, which at least one output opening 151 may be arranged off centered with respect to the symmetry axis A of the first lumen 110. The fact that the at least one output opening 151 is arranged off centered with respect to the symmetry axis A of the first lumen 110 means that the first nozzle head 150 will be caused to move within the abscess 130 due to fluid being ejected asymmetrically through the at least one opening 151. The different examples of number, size and positioning of the off center arranged output openings 151 shown in Figs. 2, 6B and 6C, respectively, are non-limiting examples shown for illustrational purposes only. As is evident to a person skilled in the art, many other arrangements wherein at least one output opening 151 is arranged off centered with respect to the symmetry axis A of the first lumen 110 are applicable and will provide the advantages of the embodiments presented herein. In any of these embodiments, the one or more openings may be of any suitable shape, and the size of the opening here refers to the size or area of the opening through which fluid is enabled to pass.

As further illustrated in Fig. 2, the output openings 151 need not be of the same size and/or shape. In some embodiments, the first nozzle head 150 may comprise at least two output openings 151 configured to eject the rinsing fluid 111, wherein a first output opening 151' of said at least two output openings is of a first size and a second output opening 151" of said at least two output openings is of a second size different from the first size. The size and/or shape and/or placement of the at least two output openings 151 may be selected such that the movement of the first nozzle head 150, causing the rinsing fluid 111 exiting from the first lumen 110 to be distributed in the abscess 130 in response to movements of the first nozzle head 150 resulting from the rinsing fluid 111 flowing through the first nozzle head, is optimized with regard to specific drainage needs.

In different configurations of the catheter system 100, the first lumen 110 may either be comprised in the second lumen 120, as illustrated in the side view and cross-section view in **Fig. 3A**, or the first lumen 110 and the second lumen 120 may be arranged side-by-side, as illustrated in the side view and cross-section view in **Fig. 3B**, possibly enclosed by an outer tube, or casing catheter, 180 as indicated in Fig. 3B with dashed lines.

In the tube-in-tube embodiments illustrated in Fig. 3A, the second lumen 120 may be configured to receive the first lumen 110 and the first nozzle head 150 while the second lumen 120 is in place inside the abscess 130. To enable this, the dimensions of the first nozzle head 150 must of course be selected such that it is enabled to pass through the second lumen 120. In other words, according to one or more embodiment, the first nozzle head 150 is structured to have appropriate size (i.e. very narrow diameter) and shape to be insertable into a tubing system, specifically into the second lumen 120 or an outer casing 180 enclosing the first and second lumens 110, 120 in case the first and second lumens 110, 120 are arranged side-by-side.

According to any of the embodiments presented herein, the second lumen 120 may further be configured to receive and transport at least one of a tool and a cannula for deployment in the abscess 130.

Turning now to **Figs. 2****,** **4** **and** **5**, the catheter system 100 may advantageously be connected to at least one, preferably both, of an internal fixation device 160 and an external fixation device 170. An internal fixation device 160 is schematically illustrated in Figs. 2, 4 and 5, and an external fixation device 170 is schematically illustrated in Figs. 4 and 5. The internal fixation device 160 may be any suitable fixation device configured to fixate the catheter system 100, in particular the outer tube being either the second lumen 120 or an outer casing 180 enclosing both the first and second lumens 110, 120, to the inside of the abscess 150. In a non-limiting example the internal fixation device 160 is a fixation balloon. The external fixation device 170 may be any suitable fixation device configured to fixate the catheter system 100, in particular the outer tube being either the second lumen 120 or an outer casing 180 enclosing both the first and second lumens 110, 120, to the skin 400 of the patient. Due to the fixation, the risk of the catheter system 100 or any part of it dislodging during use is advantageously greatly reduced. Furthermore, possibilities to decrease the necessity of several radiological procedures are introduced due to the better fixation of the catheter system 100 (internal and/or external fixation of the outer tube) in the embodiments illustrated in Fig. 3A, since the tube-in-tube system firstly renders any clogging problem less likely to happen, and secondly, if clogging occurs, the inner tube can be changed without the assistance of a radiologist. The abscess drainage will thereby be more efficient and the need of multiple drains is less likely.

In different embodiments the catheter device may be a based on a tube-over-tube, or tube-in-tube, principle, as illustrated in Figure 3A, or a tube-by-tube positioning wherein the two tubes (the lumen for drainage and the lumen for irrigation) are placed adjacent to each other, possibly within an outer casing 180, as illustrated in Figure 3B.

In the tube-in-tube scenario the first lumen 110 may in some embodiments, once inserted into the second lumen 120, be fixated to the second lumen 120 via an additional fixation device. The additional fixation device may for example be located at or near the location where the first lumen 110 is inserted into the outer lumen 120.

The catheter system 100 may be a single use system.

Turning again to **Fig. 4**, there is shown an abscess draining device 200 comprising a catheter system 100 according to any of the embodiments presented herein and a pump system 140 connected to a respective proximal end 112, 122 of the first and second lumens 110, 120. The pump system 140 is configured to pump the rinsing fluid 111 into the abscess 130 via an applied overpressure and further configured to suck abscess fluid 121 out from the abscess 130 via an applied under pressure. Fig. 4 further illustrates, for ease of understanding, a rinsing fluid container 113 for rinsing fluid to be introduced into the catheter system 100 via the pump system 140, and an abscess fluid container 123 for receiving abscess fluid exiting from the catheter system 100 via the pump system 140.

According to one or more embodiment, there is provided a kit for abscess draining comprising a catheter system 100 according to any of the embodiments presented herein and a kit package configured to enclose the catheter system. The kit may further comprise a pump system according to any of the embodiments presented herein, the pump system being connected to a respective proximal end 112, 122 of the first and second lumens 110, 120 and being configured to pump the rinsing fluid 111 into the abscess 130 via an applied overpressure and suck abscess fluid 121 out from the abscess 130 via an applied under pressure, In these embodiments, the kit package is further configured to enclose the pump system 140.

In some embodiments, control of the pressure of the rinsing fluid 111 into and the abscess fluid 121 out of the abscess 130 during operation is further advantageously enabled. In these embodiments, the abscess draining system 200 further comprises a control unit (not shown in the figures) configured to control the pump system 140 to automatically regulate the flow/pressure in the first lumen 110 and the second lumen 120 to achieve optimal rinsing/flushing and clearance/abscess fluid suction rates to and from the abscess 130. In some embodiments, the regulation may be based on performing continuous measurement of the flow or volume of fluid passing into and out of the abscess 130 and feeding the measurements to the control unit, whereby the control unit is configured to adjust the over pressure pumping the rinsing fluid 111 into the abscess 130 via the first lumen 110, and the under pressure sucking the abscess fluid 121 out via the second lumen 120, in such a way that the pressure within the abscess 130 stays as constant as possible during the abscess draining and rinsing process. An advantage of achieving regulation of the pressure inside the cavity so that it will not become too high (or too low), is that the risk of complication with bacteremia and septicemia is reduced. Also, the pressure control ensures that the abscess does not collapse before the rinsing is done, which of course improves the rinsing efficiency and reduces the risk of the abscess reoccurring at a later time. This will optimize the healing (collapse) of the abscess.

In some embodiments of the invention, the solution also enables continuous analysis of the content of the drained fluid, whereby the amount and turbidity of the drained fluid can be used to decide when to remove the catheters in a more accurate manner than when a single lumen catheter is used.

## Claims

1. A catheter system (100) for draining an abscess comprising:
- first and second lumens (110, 120), the first lumen (110) being configured to transport rinsing fluid (111) into an abscess (130) and the second lumen (120) being configured to transport abscess fluid (121) out from the abscess (130), a respective proximal end (112, 122) of the first and second lumens (110, 120) being configured to be connected to a pump system (140) so as to enable the pump system (140) to pump the rinsing fluid (111) into the abscess (130) via an overpressure applied to the first lumen (110) and suck abscess fluid (121) out from the abscess (130) via an under pressure applied to the second lumen (120); and
- a first nozzle head (150) attached to a distal end (113) of the first lumen (110), which first nozzle head (150) is configured to cause the rinsing fluid (111) exiting from the first lumen (110) to be distributed in the abscess (130) in response to movements of the first nozzle head (150) resulting from the rinsing fluid (111) flowing through the first nozzle head (150),
**characterized in that**
the first nozzle head (150) is tiltable around an axis being orthogonal to a symmetry axis (A) of the first lumen (110),
wherein the movements of the first nozzle head (150) resulting from the rinsing fluid (111) flowing through the first nozzle head (150) comprise tilting around an axis being orthogonal to a symmetry axis (A) of the first lumen (110).

2. The catheter system (100) according to claim 1, wherein the first nozzle head (150) is further configured to bend or is rotatable around an axis being parallel to the symmetry axis (A) of the first lumen (110), wherein the movements of the of the first nozzle head (150) resulting from the rinsing fluid (111) flowing through the first nozzle head (150) comprise bending or rotation around an axis being parallel to the symmetry axis (A) of the first lumen (110).

3. The catheter system (100) according to claim 1 or 2, comprising a joint (152) or other connecting means connecting the distal end (113) of the first lumen (110) to the first nozzle head (150), wherein the joint (152) or other connecting means is configured to enable tilting or rotation between the distal end (113) of the first lumen (110) to the first nozzle head (150).

4. The catheter system (100) according to any one of the preceding claims, wherein at least a part of the first nozzle head 150 comprises a flexible material causing it to bend in response to the rinsing fluid (111) flowing through the first nozzle head (150).

5. The catheter system (100) according to claim 4, wherein the first nozzle head (150) comprises at least one flexible section (153), each of the at least one flexible section (153) comprising a flexible material or being structured in a manner allowing it to bend, tilt or rotate, thereby moveably connecting the parts of the first nozzle head (150) that extend distally and proximally, respectively, from the flexible section (153).

6. The catheter system (100) according to any one of the preceding claims, wherein the first nozzle head (150) is elongated in shape.

7. The catheter system (100) according to any one of the preceding claims, wherein the first nozzle head (150) comprises at least one output opening (151) configured to eject the rinsing fluid (111), which at least one output opening (151) is arranged off centered with respect to the symmetry axis (A) of the first lumen (110).

8. The catheter system (100) according to claim 7, wherein the first nozzle head (150) comprises at least two output openings configured to eject the rinsing fluid (111), wherein a first output opening (151') of said at least two output openings is of a first size and a second output opening (151") of said at least two output openings is of a second size different from the first size.

9. The catheter system (100) according to any one of the preceding claims, wherein the first lumen (110) is comprised in the second lumen (120).

10. The catheter system (100) according to claim 9, wherein the second lumen (120) is configured to receive the first lumen (110) and the first nozzle head (150) while the second lumen (120) is in place inside the abscess (130).

11. The catheter system (100) according to claim 10, wherein the second lumen (120) is configured to receive and transport at least one of a tool and a cannula for deployment in the abscess (130).

12. The catheter system (100) according to any one of the claims 9 to 11, wherein the catheter system (100) is connected to at least one of an internal fixation device (160) and an external fixation device (170).

13. An abscess draining device (200) comprising:
- a catheter system (100) according to any of the claims 1 to 12; and
- a pump system (140) connected to a respective proximal end (112, 122) of the first and second lumens (110, 120), the pump system (140) being configured to pump the rinsing fluid (111) into the abscess (130) via an applied overpressure and suck abscess fluid (121) out from the abscess (130) via an applied under pressure.

14. A kit (300) for abscess draining comprising: a catheter system (100) according to any of the claims 1 to 12; and a kit package (301) configured to enclose the catheter system (100).

15. The kit (300) according to claim 14, further comprising a pump system (140) connected to a respective proximal end (112, 122) of the first and second lumens (110, 120), the pump system (140) being configured to pump the rinsing fluid (111) into the abscess (130) via an applied overpressure and suck abscess fluid (121) out from the abscess (130) via an applied under pressure, wherein the kit package (501) is further configured to enclose the pump system (140).

## Patentansprüche

1. Kathetersystem (100) zur Drainage eines Abszesses, aufweisend:
- ein erstes und ein zweites Lumen (110, 120), wobei das erste Lumen (110) konfiguriert ist, Spülfluid (111) in einen Abszess (130) zu transportieren, und das zweite Lumen (120) konfiguriert ist, Abszessfluid (121) aus dem Abszess (130) heraus zu transportieren, wobei ein jeweiliges proximales Ende (112, 122) des ersten und des zweiten Lumens (110, 120) konfiguriert ist, mit einem Pumpensystem (140) so verbunden zu sein, dass es dem Pumpensystem (140) ermöglicht wird, das Spülfluid (111) in den Abszess (130) über einen Überdruck zu pumpen, der auf das erste Lumen (110) aufgebracht wird, und Abszessfluid (121) aus dem Abszess (130) über einen Unterdruck abzusaugen, der auf das zweite Lumen (120) aufgebracht wird; und
- einen ersten Düsenkopf (150), der an einem distalen Ende (113) des ersten Lumens (110) angebracht ist, wobei der erste Düsenkopf (150) konfiguriert ist, das aus dem ersten Lumen (110) austretende Spülfluid (111) zu veranlassen, als Reaktion auf Bewegungen des ersten Düsenkopfs (150), die daraus resultieren, dass das Spülfluid (111) durch den ersten Düsenkopf (150) strömt, in dem Abszess (130) verteilt zu werden,
**dadurch gekennzeichnet, dass**
der erste Düsenkopf (150) um eine Achse kippbar ist, die orthogonal zu einer Symmetrieachse (A) des ersten Lumens (110) verläuft,
wobei die Bewegungen des ersten Düsenkopfs (150), die daraus resultieren, dass das Spülfluid (111) durch den ersten Düsenkopf (150) strömt, ein Kippen um eine Achse umfassen, die orthogonal zu einer Symmetrieachse (A) des ersten Lumens (110) verläuft.

2. Kathetersystem (100) nach Anspruch 1, wobei der erste Düsenkopf (150) ferner konfiguriert ist, sich um eine Achse zu biegen, oder um diese drehbar ist, die parallel zu der Symmetrieachse (A) des ersten Lumens (110) verläuft, wobei die Bewegungen des ersten Düsenkopfs (150), die daraus resultieren, dass das Spülfluid (111) durch den ersten Düsenkopf (150) strömt, eine Biegung oder Drehung um eine Achse umfassen, die parallel zu der Symmetrieachse (A) des ersten Lumens (110) verläuft.

3. Kathetersystem (100) nach Anspruch 1 oder 2, aufweisend ein Gelenk (152) oder ein anderes Verbindungsmittel, welches das distale Ende (113) des ersten Lumens (110) mit dem ersten Düsenkopf (150) verbindet, wobei das Gelenk (152) oder das andere Verbindungsmittel konfiguriert ist, ein Kippen oder eine Drehung zwischen dem distalen Ende (113) des ersten Lumens (110) und dem ersten Düsenkopf (150) zu ermöglichen.

4. Kathetersystem (100) nach einem der vorhergehenden Ansprüche, wobei zumindest ein Teil des ersten Düsenkopfs (150) ein flexibles Material aufweist, das ihn veranlasst, sich als Reaktion darauf zu biegen, dass das Spülfluid (111) durch den ersten Düsenkopf (150) strömt.

5. Kathetersystem (100) nach Anspruch 4, wobei der erste Düsenkopf (150) mindestens einen flexiblen Abschnitt (153) aufweist, wobei jeder des mindestens einen flexiblen Abschnitts (153) ein flexibles Material aufweist oder so strukturiert ist, dass er sich biegen, kippen oder drehen kann, wodurch die Teile des ersten Düsenkopfs (150), die sich distal bzw. proximal von dem flexiblen Abschnitt (153) erstrecken, bewegbar verbunden sind.

6. Kathetersystem (100) nach einem der vorhergehenden Ansprüche, wobei der erste Düsenkopf (150) eine längliche Form hat.

7. Kathetersystem (100) nach einem der vorhergehenden Ansprüche, wobei der erste Düsenkopf (150) mindestens eine Ausgangsöffnung (151) aufweist, die konfiguriert ist, das Spülfluid (111) auszustoßen, wobei die mindestens eine Ausgangsöffnung (151) in Bezug auf die Symmetrieachse (A) des ersten Lumens (110) außermittig angeordnet ist.

8. Kathetersystem (100) nach Anspruch 7, wobei der erste Düsenkopf (150) mindestens zwei Ausgangsöffnungen aufweist, die konfiguriert sind, das Spülfluid (111) auszustoßen, wobei eine erste Ausgangsöffnung (151') der mindestens zwei Ausgangsöffnungen eine erste Größe hat und eine zweite Ausgangsöffnung (151") der mindestens zwei Ausgangsöffnungen eine zweite Größe hat, die sich von der ersten Größe unterscheidet.

9. Kathetersystem (100) nach einem der vorhergehenden Ansprüche, wobei das erste Lumen (110) in dem zweiten Lumen (120) enthalten ist.

10. Kathetersystem (100) nach Anspruch 9, wobei das zweite Lumen (120) konfiguriert ist, das erste Lumen (110) und den ersten Düsenkopf (150) aufzunehmen, während sich das zweite Lumen (120) innerhalb des Abszesses (130) befindet.

11. Kathetersystem (100) nach Anspruch 10, wobei das zweite Lumen (120) konfiguriert ist, ein Werkzeug und/oder eine Kanüle zum Einsatz in dem Abszess (130) aufzunehmen und zu transportieren.

12. Kathetersystem (100) nach einem der Ansprüche 9 bis 11, wobei das Kathetersystem (100) mit einer internen Fixierungsvorrichtung (160) und/oder einer externen Fixierungsvorrichtung (170) verbunden ist.

13. Vorrichtung zur Drainage von Abszessen (200), aufweisend:
- ein Kathetersystem (100) nach einem der Ansprüche 1 bis 12; und
- ein Pumpensystem (140), das mit einem jeweiligen proximalen Ende (112, 122) des ersten und des zweiten Lumens (110, 120) verbunden ist, wobei das Pumpensystem (140) konfiguriert ist, das Spülfluid (111) über einen aufgebrachten Überdruck in den Abszess (130) zu pumpen und Abszessfluid (121) über einen aufgebrachten Unterdruck aus dem Abszess (130) abzusaugen.

14. Kit (300) zur Drainage von Abszessen, aufweisend: ein Kathetersystem (100) nach einem der Ansprüche 1 bis 12; und eine Kit-Umhüllung (301), die konfiguriert ist, das Kathetersystem (100) zu umschließen.

15. Kit (300) nach Anspruch 14, ferner aufweisend ein Pumpensystem (140), das mit einem jeweiligen proximalen Ende (112, 122) des ersten und des zweiten Lumens (110, 120) verbunden ist, wobei das Pumpensystem (140) konfiguriert ist, das Spülfluid (111) über einen aufgebrachten Überdruck in den Abszess (130) zu pumpen und Abszessfluid (121) über einen aufgebrachten Unterdruck aus dem Abszess (130) abzusaugen, wobei die Kit-Umhüllung (501) ferner konfiguriert ist, das Pumpensystem (140) zu umschließen.

## Revendications

1. Système de cathéter (100) pour drainer un abcès comprenant :
- des première et deuxième lumières (110, 120), la première lumière (110) étant configurée pour transporter un fluide de rinçage (111) dans un abcès (130) et la deuxième lumière (120) étant configurée pour transporter le fluide d'abcès (121) hors de l'abcès (130), une extrémité proximale (112, 122) respective des première et deuxième lumières (110, 120) étant configurée pour être reliée à un système de pompe (140) de manière à permettre au système de pompe (140) de pomper le fluide de rinçage (111) dans l'abcès (130) par le biais d'une surpression appliquée à la première lumière (110) et aspirer le fluide d'abcès (121) hors de l'abcès (130) par le biais d'une sous-pression appliquée à la deuxième lumière (120) ; et
- une première tête de buse (150) fixée à une extrémité distale (113) de la première lumière (110), laquelle première tête de buse (150) est configurée pour amener le fluide de rinçage (111) sortant de la première lumière (110) à être distribué dans l'abcès (130) en réponse aux mouvements de la première tête de buse (150) résultant de l'écoulement du fluide de rinçage (111) à travers la première tête de buse (150),
**caractérisé en ce que**
la première tête de buse (150) peut basculer autour d'un axe perpendiculaire à un axe de symétrie (A) de la première lumière (110),
dans lequel les mouvements de la première tête de buse (150) résultant de l'écoulement du fluide de rinçage (111) à travers la première tête de buse (150) comprennent le basculement autour d'un axe perpendiculaire à un axe de symétrie (A) de la première lumière (110).

2. Système de cathéter (100) selon la revendication 1, dans lequel la première tête de buse (150) est en outre configurée pour fléchir ou peut tourner autour d'un axe parallèle à l'axe de symétrie (A) de la première lumière (110), dans lequel les mouvements de la de la première tête de buse (150) résultant de l'écoulement du fluide de rinçage (111) à travers la première tête de buse (150) comprennent le fléchissement ou la rotation autour d'un axe parallèle à l'axe de symétrie (A) de la première lumière (110).

3. Système de cathéter (100) selon la revendication 1 ou 2, comprenant un joint (152) ou un autre moyen de liaison reliant l'extrémité distale (113) de la première lumière (110) à la première tête de buse (150), dans lequel le joint (152) ou l'autre moyen de liaison est configuré pour permettre un basculement ou une rotation entre l'extrémité distale (113) de la première lumière (110) par rapport à la première tête de buse (150).

4. Système de cathéter (100) selon l'une quelconque des revendications précédentes, dans lequel au moins une partie de la première tête de buse (150) comprend un matériau flexible l'amenant à fléchir en réponse à l'écoulement du fluide de rinçage (111) à travers la première tête de buse (150).

5. Système de cathéter (100) selon la revendication 4, dans lequel la première tête de buse (150) comprend au moins une section flexible (153), chacune de la au moins une section flexible (153) comprenant un matériau flexible ou étant structurée d'une manière lui permettant de fléchir, s'incliner ou tourner, reliant ainsi de manière mobile les parties de la première tête de buse (150) qui s'étendent de manière distale et proximale, respectivement, à partir de la section flexible (153).

6. Système de cathéter (100) selon l'une quelconque des revendications précédentes, dans lequel la première tête de buse (150) est de forme allongée.

7. Système de cathéter (100) selon l'une quelconque des revendications précédentes, dans lequel la première tête de buse (150) comprend au moins une ouverture de sortie (151) configurée pour éjecter le fluide de rinçage (111), laquelle au moins une ouverture de sortie (151) est agencée de manière décentrée par rapport à l'axe de symétrie (A) de la première lumière (110).

8. Système de cathéter (100) selon la revendication 7, dans lequel la première tête de buse (150) comprend au moins deux ouvertures de sortie configurées pour éjecter le fluide de rinçage (111), dans lequel une première ouverture de sortie (151') desdites au moins deux ouvertures de sortie est d'une première taille et une deuxième ouverture de sortie (151") desdites au moins deux ouvertures de sortie est d'une deuxième taille différente de la première taille.

9. Système de cathéter (100) selon l'une quelconque des revendications précédentes, dans lequel la première lumière (110) est comprise dans la deuxième lumière (120).

10. Système de cathéter (100) selon la revendication 9, dans lequel la deuxième lumière (120) est configurée pour recevoir la première lumière (110) et la première tête de buse (150) pendant que la deuxième lumière (120) est en place à l'intérieur de l'abcès (130).

11. Système de cathéter (100) selon la revendication 10, dans lequel la deuxième lumière (120) est configurée pour recevoir et transporter au moins l'un d'un outil et d'une canule pour un déploiement dans l'abcès (130).

12. Système de cathéter (100) selon l'une quelconque des revendications 9 à 11, dans lequel le système de cathéter (100) est relié à au moins l'un d'un dispositif de fixation interne (160) et d'un dispositif de fixation externe (170).

13. Dispositif de drainage d'abcès (200) comprenant :
- un système de cathéter (100) selon l'une quelconque des revendications 1 à 12 ; et
- un système de pompe (140) relié à une extrémité proximale (112, 122) respective des première et deuxième lumières (110, 120), le système de pompe (140) étant configuré pour pomper le fluide de rinçage (111) dans l'abcès (130) par le biais d'une surpression appliquée et aspirer le fluide d'abcès (121) hors de l'abcès (130) par le biais d'une sous-pression appliquée.

14. Kit (300) pour drainage d'abcès comprenant : un système de cathéter (100) selon l'une quelconque des revendications 1 à 12 ; et un emballage de kit (301) configuré pour renfermer le système de cathéter (100).

15. Kit (300) selon la revendication 14, comprenant en outre un système de pompe (140) relié à une extrémité proximale (112, 122) respective des première et deuxième lumières (110, 120), le système de pompe (140) étant configuré pour pomper le fluide de rinçage (111) dans l'abcès (130) par le biais d'une surpression appliquée et aspirer le fluide d'abcès (121) hors de l'abcès (130) par le biais d'une sous-pression appliquée, dans lequel l'emballage de kit (501) est en outre configuré pour renfermer le système de pompe (140).
